# EUROPEAN PATENT APPLICATION

(11) **EP 0 737 666 A1**
(43) Date of publication of application: **16.10.1996**
(21) Application number: 96200974.2
(22) Date of filing: 09.04.1996
(51) Int. Cl.: C07C 209/62, C07C 253/20

(54) **Depolymerization of polyamides**

(30) Priority: 14.04.1995 BE 9500347
(71) Applicant: DSM N.V., 6411 TE Heerlen (NL)
(72) Inventor: Hendrix, Jan Agnes Jozef, 6125 CB Obbicht (NL); Booij, Martin, 6151 DH Munstergeleen (NL); Frentzen, Yvonne Helene, 5921 ES Venlo (NL)

(57) **Abstract**

Process for the depolymerization of one or more polyamides in the presence of at least a nitrogen-containing compound, the depolymerization taking place at a pressure between 0.5 and 5 atm.

As a result a better selectivity towards the monomeric components of polyamides is obtained.

## Description

The invention relates to a process for the depolymerization of one or more polyamides in the presence of at least a nitrogen-containing compound.

Such a process is known from US-A-5.302.756. Said patent publication describes the depolymerization of nylon 6.6 or a mixture of nylon 6 and nylon 6.6 with the aid of ammonia at high pressures, whether or not in the presence of a phosphate catalyst, resulting in the formation of monomeric components.

The drawback of this known process is the low selectivity towards the monomeric components of the polyamides. For nylon 6 these are caprolactam and caprolactam precursors. Precursors are understood to be those compounds that can be used for the preparation of the polyamides without first having to be converted, such as aminocaproic acid.

Polyamides (production waste, for example) have been recycled for some 40 years already. This takes place in particular at the polyamide producers' and polyamide fibre spinners'. In the past few years product recycling has increasingly been taking place at carpet manufacturers'. Depolymerization of polyamides is of relevance to polyamide products in general and to carpets in which polyamides are processed in particular. In the future it will become increasingly important to recycle carpet waste, both industrial carpet waste and the so-called post-consumer carpet waste, in an economic manner, a.o. because landfill capacity is nearing maximum utilization. Of course, the commercial feasibility of carpet waste recycling is directly dependent on the economic/technical capability of converting the polyamide part of these carpets into monomeric components which can -preferably immediately, that is, without further conversion reactions- be reused. For this reason there is a need for further improvement of processes for carpet waste recycling.

The aim of the invention is to provide a process that yields a better selectivity towards the monomeric components of polyamides.

This aim is achieved in that depolymerization takes place at a pressure between 0.5 and 5 atm. Preferably, depolymerization takes place at a pressure between 0.9 and 3 atm.

Not only is a higher selectivity obtained, but also additional investment costs for a depolymerization facility needed for high-pressure depolymerization, are prevented. This makes the process according to the invention very attractive from an economic point of view. In US-A-5.302.756, by contrast, the depolymerization is carried out at pressures of at least 7 atm. Its examples describe pressures varying from 30 to 300 atm. There is no indication at all in said patent publication that depolymerization carried out at a low pressure would be possible and would result in such high selectivities. US-A-5.302.756 teaches away from the present invention. Table 3 of US-A-5.302.756 illustrates that higher pressures are preferred, because the higher the pressure, the higher the overall yield.

The nitrogen-containing compounds that are suitable for the depolymerization reaction are, for example, NH₃, (cyclo)aliphatic amines, (cyclo)aliphatic diamines, (cyclo)aliphatic polyamines, with the aliphatic group comprising 1-20 carbon atoms; aromatic amines, aromatic diamines and/or aromatic polyamines. Examples of such compounds are aminomethane, aminoethane, aminopropane, aminobutane, aminopentane, aminohexane, aminododecane, 1,2-diaminoethane, 1,3-diaminopropane, 1,3-diaminobutane, 1,2-diaminobutane, 1,4-diaminobutane (DAB), 1,4-diaminohexane (HDA), 1,6-diaminohexane (HMDA), dimethylamine, diethylamine, dipropylamine, trimethylamine, triethylamine, tripropylamine, (di)cyclohexylamine, (di)aminocyclohexane, 4-aminomethyl-1,8-diaminooctane, 3-aminomethyl-1,6-diaminohexane, bisethylene triamine, bistetramethylene triamine, bishexamethylene triamine, amino(alkyl)-substituted piperidines, amino(alkyl)-substituted aromatics. Preferably, a nitrogen-containing compound is used that has a boiling point below that of caprolactam (270°C). In particular, NH₃, aminoethane, aminopropane, diaminoethane, diaminopropane, diaminobutane or diaminohexane is used as nitrogen-containing compound.

The amount of nitrogen-containing compound is generally at least 1 mole per mole of amide group in the polyamide to be depolymerized. Preferably, more than 2 moles per mole of amide group is used. In general, the amount will be smaller than 250 moles of nitrogen-containing compound per mole of amide group, preferably smaller than 100.

To influence the reaction velocity and the selectivity, the depolymerization reaction is preferably carried out in the presence of Lewis acids, e.g. Al₂O₃ or SiO₂, Brønsted acids, e.g. H₃PO₄ or H₃BO₃, or salts thereof, for example ammonium salts, oligomers of nylon 6 or nylon 6,6. These compounds are present in an amount of 0.1-20 wt.% relative to the nitrogen-containing compound, preferably 1-10 wt.%. It is also possible to influence the reaction velocity and the selectivity through complexing of the nitrogen-containing compound with metal compounds, e.g. copper salts, cobalt salts. The metal compound is then present in an amount of 0.1-10 wt.% relative to the nitrogen-containing compound, preferably 0.5-5 wt.%.

The depolymerization can also be carried out in the presence of water next to the presence of the nitrogen-containing compound. The amount of water is at most 50 wt.% relative to the polyamide, preferably at most 20 wt.%, in particular at most 10 wt.%. The polyamide usually contains some water; the process being generally carried out in the presence of more than 1 wt.% water. Although it is known from JP-A-46-31541 to depolymerize polyamides in the presence of ammonia and water, according to the process described in this Japanese patent specification the depolymerization is carried out at very high pressures.

The temperature at which the process for the depolymerization according to the present invention can be carried out lies between 200 and 400°C. Preferably, the temperature lies between 250 and 350°C.

The depolymerization reaction can be carried out by contacting the nitrogen-containing compound with the polyamide, which first has been molten, The polyamide is then split up into its monomeric components. These monomeric components are preferably removed via the gas phase, together with the nitrogen-containing compound.

The nitrogen-containing compounds can be separated from the monomeric components using techniques that are known per se. Examples of such techniques are steam distillation and vacuum distillation. Distillation can take place in one or more steps. These nitrogen-containing compounds can then directly be returned to the depolymerization reactor and be reused. The monomeric components and their precursors can subsequently be purified by means of, for example, distillation (steam or vacuum distillation), recrystallization, or other customary purification techniques.

The process according to the present invention can excellently be used for processing of polyamide, products made of or containing polyamides, in particular industrial carpet waste and post-consumer carpet waste. Preferably, the carpet is first subjected to mechanical size reduction, for example by grinding, chopping and/or cutting. The larger part of the non-polyamide components of the carpet, for example latex (which may be filled with CaCO₃), jute and/or polypropylene, can be separated from the polyamide part of the carpet in one or more separation steps. However, the carpet shreds can also be fed to the reactor as such. The process according to the invention achieves good results at polyamide levels in the reactor of even as low as 1%, in combination with other components.

### Example I

40.0 g of nylon 6 was introduced into a 2-litre CrNi steel reactor while a small nitrogen flow was being passed through at 290°C. After about 2 minutes the supply of ammonia gas was started (285 Nl/h). This was done via a spiral line discharging into a feed opening at the centre of the reactor bottom. Both the gas feed line and the reactor were heated by means of a salt bath at a temperature of 350°C. The depolymerization was carried out at a temperature of 330°C. The pressure was 1 atm.

A misty off-gas was formed (aerosol), which was immediately removed from the reactor. At colder spots condensate separated from the aerosol, which was deposited on the walls of the discharge tubing.

The aerosol, the condensate and the deposit that had separated off from the aerosol were all collected in water. Thus, after 2 hours a total amount of 8.3 g of product had been recovered in water, consisting of:
83.3 wt.% caprolactam
12.9 wt.% aminocaproic acid
2.0 wt.% aminocapronamide
4.3% aminocapronitrile.
The residue in the reactor had a white colour, which means no tar formation or degradation had occurred. This implies that the residue can again be subjected to a depolymerization reaction. The selectivity towards nylon 6 monomer was calculated as follows:$\text{selectivity =} \frac{\text{mmole caprolactam + mmole aminocaproic acid}}{\text{mmole recovered product}} \text{= 100%}$ The selectivity of this depolymerization was 97%. After purification caprolactam and aminocaproic acid were directly available for reuse. Aminocapronamide and aminocapronitrile can be converted to caprolactam and aminocaproic acid by means of hydrolysis and cyclization reactions.

### Example II

Into a stirred 2-litre double-walled CrNi steel reactor 45 g of nylon 6 was introduced as well as 4.5 g of ammonium dihydrogen phosphate, while a small nitrogen flow was being passed through at 310°C. After about 2 minutes the supply of ammonia gas was started (194 Nl/h). This was done via a jacketed feed line discharging into a feed opening at the centre of the reactor bottom. Both the feed line and the reactor were heated by means of oil at a temperature of 330°C. The depolymerization was carried out at a temperature of 315°C. The pressure was 1 atmosphere.

A misty off-gas was formed (aerosol), which was immediately removed from the reactor.

The double-walled cover of the reactor (oil-heated) as well as the gas discharge line were heated at a temperature of 250°C.

The resulting gas flow was condensed with the help of a spiral condenser heated at 80°C. Thus, after 1 hour a total amount of 38.4 g of product was recovered, which consisted of:
83.4 wt.% caprolactam
<0.1 wt.% aminocaproic acid
0.8 wt.% aminocapronamide
11.8 wt.% aminocapronitrile
The residue in the reactor was clear and of a light brown colour, which indicates only little tar formation and/or degradation had occurred. The selectivity was 87%.

### Comparative experiment A

40 g of nylon 6 was depolymerized in a 2-litre autoclave in the presence of 1.3 g of diammonium hydrogen phosphate. 11 ml/min of liquid ammonia was fed to the autoclave at a temperature of 320°C. A pressure valve was used to control the autoclave pressure at 68 atm.
The volatile reaction products left the autoclave via the off-gas.
The monomers yield was as follows:
37 wt.% caprolactam
0 wt.% aminocaproic acid
50 wt.% aminocapronamide
1 wt.% aminocapronitrile
The selectivity was 45%.

### Example III

### Part A

This Example was carried out as Example II, only now 1.5 g ammonium dihydrogen phosphate was used.

After 1 hour a total amount of 30.5 g of product had been recovered in 5 fractions, these fractions being collected after 10, 20, 30, 40 and 60 minutes. This 30.5 g consisted of:
79.6 wt.% caprolactam
0.5 wt.% aminocaproic acid
0.4 wt.% aminocapronamide
15.0 wt.% aminocapronitrile
The selectivity was 84%.
In Table 1 the weight and the composition of each fraction is given.

**Table 1**

| Fraction (no) | Weight (g) | Caprolactam (mmole) | Aminocapronamide (mmole) | Aminocaproic acid (mmole) | Aminocapronitrile (mmole) |
|---|---|---|---|---|---|
| 1 | 12.0 | 91.6 | 0.2 | 0.3 | 7.0 |
| 2 | 8.5 | 61.5 | 0.2 | 0.3 | 11.8 |
| 3 | 5.6 | 38.0 | 0.2 | 0.3 | 10.0 |
| 4 | 2.7 | 16.3 | 0.2 | 0.2 | 6.1 |
| 5 | 1.7 | 7.6 | 0.2 | 0.1 | 5.9 |

### Part B

Immediately after the depolymerization described in part A, the depolymerization was repeated, without the residue having been removed from the reactor. Again 45 g of nylon 6 was introduced into the reactor while a small nitrogen flow was passed through at 310°C, but no fresh additional ammonium dihydrogen phosphate was added.

After 50 minutes a total amount of 39.7 g of product was recovered in four fractions, these fractions being collected after 10, 20, 30 and 50 minutes. This 37.9 g consisted of:
76.7 wt.% caprolactam
0.4 wt.% aminocaproic acid
0.4 wt.% aminocapronamide
15.0 wt.% aminocapronitrile
The selectivity was 84%.
In Table 2 the weight and composition of each fraction is given.

**Table 2**

| Fraction (no) | Weight (g) | Caprolactam (mmole) | Aminocapronamide (mmole) | Aminocaproic acid (mmole) | Aminocapronitrile (mmole) |
|---|---|---|---|---|---|
| 1 | 15.4 | 114.8 | 0.4 | 0.4 | 12.4 |
| 2 | 10.6 | 75.4 | 0.3 | 0.3 | 15.5 |
| 3 | 6.9 | 44.6 | 0.2 | 0.2 | 12.8 |
| 4 | 6.2 | 34.6 | 0.3 | 0.3 | 12.5 |

From the results of part A and part B of this Example it becomes clear that both the production of the monomers and the selectivity towards the monomers do not differ significantly. This illustrates that the phosphate compound can be recycled. This becomes clear from the selectivity results of part A and part B of this Example.
In addition, both Tables 1 and 2 illustrate that the selectivity and velocity of the depolymerization reaction towards nylon 6 monomers (caprolactam and aminocaproic acid) are remarkably better in the first fraction compared with the last fraction. This implies that continuous operation of the depolymerization process according to the present invention would significantly improve the overall selectivity towards nylon 6 monomers.

### Example IV

This Example was carried out as described in Example II. Instead of ammonia, aminopropane (132 g/h) was used as nitrogen-containing compound. The aminopropane was dosed by evaporating liquid aminopropane in a jacketed feed line discharging into the feed opening at the bottom of the reactor.

After 4 hours 397.4 g of product was recovered, consisting of:
1.6 wt.% caprolactam
<0.1 wt.% aminocaproic acid
<0.1 wt.% aminocapronamide
<0.1 wt.% aminocapronitrile
The residue in the reactor had a white colour. The selectivity was > 95%.
From this Example it can be concluded that with aminopropane high selectivities can be obtained.

### Example V

This Example was carried out as described in Example II. Instead of ammonia, 132 g/h of aminopropane was used, dosing taking place as described in Example IV.

After 2 hours 207.7 g of product had been recovered in 2 fractions, these fractions being collected at the end of each hour. The 207.7 g consisted of:
15.1 wt.% caprolactam
<0.1 wt.% aminocaproic acid
<0.1 wt.% aminocapronamide
0.5 wt.% aminocapronitrile
The residue in the reactor was clear and of a light brown colour, and only little formation of tar and/or degradation had occurred.
The selectivity was 96%.
In Table 3 the weight and composition of each fraction is given.

**Table 3**

| Fraction (no) | Weight (g) | Caprolactam (mmole) | Aminocapronamide (mmole) | Aminocaproic acid (mmole) | Aminocapronitrile (mmole) |
|---|---|---|---|---|---|
| 1 | 120.2 | 271.2 | <1 | 1.2 | 7.3 |
| 2 | 87.5 | 6.0 | <1 | <1 | 2.6 |

It becomes clear from Table 3 that over 95% of the nylon 6 monomers was produced in the first hour. Again, as in Example III, it can be concluded that continuous operation of the depolymerization process according to the present invention leads to very high selectivity results.

## Claims

1. Process for the depolymerization of one or more polyamides in the presence of at least a nitrogen-containing compound, characterized in that depolymerization takes place at a pressure between 0.5 and 5 atm.

2. Process according to claim 1, characterized in that depolymerization takes place at a pressure between 0.9 and 3 atm.

3. Process according to claim 1 or claim 2, characterized in that the nitrogen-containing compound is NH₃, a (cyclo)aliphatic amine, a (cyclo)aliphatic diamine, a (cyclo)aliphatic polyamine, with the aliphatic group comprising 1-20 carbon atoms; an aromatic amine, an aromatic diamine and/or an aromatic polyamine.

4. Process according to claim 3, characterized in that the nitrogen-containing compound has a boiling point that is lower than the boiling point of caprolactam.

5. Process according to claim 3, characterized in that the nitrogen-containing compound is NH₃, aminoethane, aminopropane, diaminoethane, diaminopropane, diaminobutane or diaminohexane.

6. Process according to any one of claims 1-5, characterized in that the amount of nitrogen-containing compound is at least 1 mole per mole of amide group in the polyamide to be depolymerized.

7. Process according to any one of claims 1-6, characterized in that one or more compounds chosen from the group formed by Lewis acids, Brønsted acids, oligomers of polyamides or metal compounds are present in the process.

8. Process according to claim 7, characterized in that the amount of the compound(s) chosen from the group formed by Lewis acids, Brønsted acids, oligomers of polyamides is 0.1-20 wt.% relative to the nitrogen-containing compound.

9. Process according to claim 7, characterized in that the amount of metal compound(s) is 0.1-10 wt.% relative to the nitrogen-containing compound.

10. Process according to claim 7, characterized in that the (metal) compound(s) can be reused.

11. Process according to any one of claims 1-10, characterized in that the depolymerization is carried out in the presence of at most 50 wt.% water relative to the polyamide(s).

12. Process according to any one of claims 1-11, characterized in that the nitrogen-containing compound is separated from caprolactam produced and is reused in the depolymerization.

13. Process according to any one of claims 1-12, characterized in that the depolymerization takes place at a temperature of between 200 and 400°C.

14. Process according to any one of claims 1-13, characterized in that polyamide-containing carpet waste is used in the depolymerization.

15. Process according to any one of claims 1-14, characterized in that the depolymerization is carried out continuously.
